# EUROPEAN PATENT APPLICATION

(11) **EP 4 610 361 A1**
(43) Date of publication of application: **03.09.2025**
(21) Application number: 23883000.4
(22) Date of filing: 19.10.2023
(51) Int. Cl.: C12N 15/85, C07K 14/78, C12N 5/0775, A61K 47/46

(54) **EXTRACELLULAR VESICLES OVEREXPRESSING FIBRONECTIN FRAGMENT PROTEIN, AND USE FOR DRUG DELIVERY THEREOF**

(30) Priority: 24.10.2022 KR 20220137524
(71) Applicant: Dongguk University Industry-Academic Cooperation Foundation, Seoul 04620 (KR)
(72) Inventor: LEE, Soo-Hong, Hanam-si Gyeonggi-do 12912 (KR); ARAI, Yoshie, Ilsandong-gu Goyang-si Gyeonggi-do 10325 (KR); KIM, Si Yeon, Jochiwon-eup Sejong 30021 (KR); KIM, Jiseong, Gwangsan-gu Gwangju 62256 (KR); BELLO, Alvin, Deogyang-gu Goyang-si Gyeonggi-do 10459 (KR)
(74) Representative: Mewburn Ellis LLP
(86) International application number: PCT/KR2023/016244
(87) International publication number: WO 2024/090893

(57) **Abstract**

The present invention relates to extracellular vesicles (EVs) overexpressing fibronectin fragment protein, and a use for drug delivery thereof. The present invention relates to a method for increasing EV delivery efficiency into cells by overexpressing a RGD domain and a Hep II domain of fibronectin on an EV membrane surface, and through the method, it was confirmed that EVs overexpressing fibronectin domains had increased EV delivery efficiency into cells compared to a control group. It was confirmed that an overexpression process through gene transduction could only improve cell transfer efficiency without affecting the size of EVs or the expression of marker genes (CD9, HSP70). The method reduces the time and effort required for EV research through effective delivery of EVs to cells, and can be used to study mechanisms between cells and as a drug delivery medium for diseases and disorders.

## Description

### Technical Field

The present disclosure relates to an extracellular vesicle (EV) on which a fibronectin fragment protein is overexpressed and a use for drug delivery thereof.

### Background Art

An EV, a vesicle that is exocytosed by cells, is formed in a lipid bilayer structure and responsible for interactions among cells. In addition, the EV contains metabolites such as proteins and nucleic acids from the cells that they originate from and thus plays an important role in signal transmission between cells. Recently, it has been gaining attention for its value as a material for treatment and regeneration as well as a drug delivery vehicle through processes such as insertion of specific substances into the EVs.

The EV is made of a lipid bilayer membrane and contains many proteins on its membrane surface. Thereamong, several papers have claimed that fibronectin and heparan sulfate on the EV surface are responsible for the interaction between cells and EVs.

Research is also being actively conducted to create EVs that deliver substances of interest and have specificity for particular tissues or cells after undergoing processes of attaching specific proteins or amino acid sequences to a membrane protein called Lamp2b that is present on the surface of EVs. As such, EVs are promising as future therapeutic drugs and mediators that will help understand the mechanisms between cells, and more research is being conducted to determine their mechanisms and exact definition.

EVs can be acquired from body fluids, serum, or cell cultures, but even though EVs take effect by entering target cells, experiments and treatments require a huge amount of body fluids, serum, and cells, which involve a tremendous amount of time, effort, and cost.

To overcome these limitations, an approach may be presented as a solution that allows cells to take up a large amount of EVs even when a small amount of EVs is treated, thereby enabling effective utilization of effects of EVs.

### Disclosure of the Invention

### Technical Goals

An object of the present disclosure is to provide an extracellular vesicle wherein a fibronectin fragment protein including a fibronectin RGD domain and a fibronectin Hep II domain is overexpressed on a membrane surface of the extracellular vesicle, and the fibronectin fragment protein may be additionally fused a Lamp2b protein.

In addition, another object of the present disclosure is to provide a use for drug delivery including the extracellular vesicle.

### Technical Solutions

To achieve the above object, the present disclosure provides an extracellular vesicle wherein a fibronectin fragment protein including a fibronectin RGD domain and a fibronectin Hep II domain is overexpressed on a membrane surface of the extracellular vesicle, and the fibronectin fragment protein may be additionally fused a Lamp2b protein.

In order to achieve the other object above, the present disclosure provides a drug delivery composition including the extracellular vesicle.

### Advantageous Effects

The present disclosure relates to an extracellular vesicle on which a fibronectin fragment protein is overexpressed and a use for drug delivery thereof, which may derive, even when a small amount of EVs is treated, the same therapeutic effects and changes as those when a large amount of EVs is treated. A RGD domain and a Hep II domain of fibronectin are responsible for an adhesive behavior of cells. Therefore, since the two domains organically increase cell adhesiveness, by overexpressing this part on the EV membrane, EVs are able to better adhere to cells so as to easily enter the cell. In other words, according to the present disclosure, as the EV has increased adhesion to cells to better enter the cells, even when a small amount of EVs is treated, the same effect is shown as when a large amount of EVs is treated, thereby reducing a lot of time, effort, and materials to be used as an effective drug delivery material.

### Brief Description of Drawings

FIG. 1 shows a sequence of an open reading frame (ORF) of a Lamp2b protein overexpression vector in which RGD and Hep II domains of fibronectin are combined.
FIG. 2(A) shows a schematic diagram of a vector capable of expressing RGD and Hep II domains of fibronectin combined with Lamp2b. FIG. 2(B) shows a schematic diagram visualizing a target protein in a vector, and FIG. 2(C) shows schematic diagrams illustrating a resulting processed EV.
FIG. 3 shows results of measuring a size and concentration of each of EVs extracted from normal MSCs and EVs extracted from transduced MSCs using a nanoparticle tracking analyzer.
FIG. 4(A) shows results of identifying expression levels of fibronectin from EVs extracted from normal MSCs and EVs extracted from transduced MSCs as well as CD9 and HSP70 which are proteins expressed from EVs via Western blot, and FIG. 4(B) shows a result of quantifying band intensities of FIG. 4(A) using the Image J program.
FIG. 5 shows images illustrating that an EV uptake rate of cells is lower when EVs are treated with fibronectin antibodies or RGD peptides compared to normal EVs.
FIG. 6 shows images illustrating that a delivery efficiency of EVs overexpressing a fibronectin domain is higher when compared to an intracellular delivery efficiency of EVs by treating MSCs with a control group with no EV treatment, normal EVs, and EVs overexpressing the fibronectin domain, respectively.
FIG. 7 shows results of identifying that fibronectin domain-overexpressing EVs (FN EVs) have significantly increased intracellular delivery efficiency in various types of cells compared to Mock EVs, which are EVs with no overexpression.
FIG. 8 shows results that an intracellular delivery efficiency is remarkably reduced in experimental group 3 treated with anti-RGD antibodies and anti-Hep II antibodies compared to a control group.
FIG. 9 shows results of identifying that an intracellular delivery efficiency of EVs is highest in experimental group 1, in which both domains are overexpressed, compared to a control group, which is an EV with no overexpression.
FIG. 10 shows an intracellular delivery efficiency of EVs, obtained by quantifying a red fluorescence intensity of FIG. 9.

### Best Mode for Carrying Out the Invention

The present disclosure aims to induce the same therapeutic effects and changes as when a large amount of EV is treated, even when a small amount of EV is treated. A RGD domain and a Hep II domain of fibronectin are responsible for an adhesive behavior of cells. Therefore, since the two domains organically increase cell adhesiveness, the present disclosure was completed by determining that EVs are able to better adhere to cells and easily enter the cell by overexpressing this part on the EV membrane.

The present disclosure provides an extracellular vesicle wherein a fibronectin fragment protein including a fibronectin RGD domain and a fibronectin Hep II domain is overexpressed on a membrane surface of the extracellular vesicle.

Preferably, the fibronectin fragment protein may be additionally fused a Lamp2b protein, but is not limited thereto.

Preferably, the fibronectin RGD domain may be represented by an amino acid sequence of SEQ ID NO: 1, the fibronectin Hep II domain may be represented by an amino acid sequence of SEQ ID NO: 2, and the Lamp2b protein may be represented by an amino acid sequence of SEQ ID NO: 3, but are not limited thereto.

Preferably, the fibronectin fragment protein and the Lamp2b protein may be linked by a linker, but are not limited thereto.

Preferably, the extracellular vesicle may have a size of 100 to 200 nm, but is not limited thereto.

The present disclosure provides a drug delivery composition including the extracellular vesicle.

Preferably, the composition may enhance a delivery efficiency of extracellular vesicles into cells.

The drug as used herein is a substance capable of inducing a desired biological or pharmacological effect by promoting or suppressing a physiological function in the body of an animal or a human, refers to a chemical or biological substance or compound suitable for administration to an animal or a human, has a preventive effect on organisms by preventing undesired biological effects such as infection prevention, alleviates conditions caused by diseases, for example, alleviate pain or infection caused as a result of diseases, and plays a role in alleviating, reducing, or completely eliminating diseases from organisms.

The drug delivery composition of the present disclosure may further include one or more pharmaceutically acceptable carriers, excipients, or diluents in addition to the extracellular vesicles.

In addition, as used herein, the term "pharmaceutically acceptable" refers to a condition that is physiologically acceptable without causing allergic reactions such as gastrointestinal disorders or dizziness or similar reactions when administered to humans.

Examples of carriers, excipients, and diluents include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methyl hydroxybenzoate, propyl hydroxybenzoate, talc, magnesium stearate, and mineral oil.

In addition, the drug delivery composition may additionally include fillers, anticoagulants, lubricants, wetting agents, fragrances, emulsifiers, and preservatives.

The drug delivery composition according to the present disclosure may be administered via various routes including orally, transdermally, subcutaneously, intravenously, or intramuscularly, and the dosage of the drug may be appropriately selected depending on various factors such as the route of administration, the age, sex, and weight of the patient, and the severity of the patient. Additionally, the drug delivery composition of the present disclosure may be administered in combination with a known compound that may enhance the desired effect of the drug.

### Modes for Carrying Out the Invention

Hereinafter, the present disclosure will be described in more detail through examples to help understanding of the present disclosure. However, examples below are provided to more completely describe the present disclosure to those with ordinary skill in the art and merely illustrate the content of the present disclosure, and the scope of the present disclosure is not limited to the following examples.

### <Experimental Example>

The following Experimental Examples are intended to provide Experimental Examples commonly applied to each Examples according to the present disclosure.

### 1. Genetic transduction for production of EVs overexpressing fibronectin domains

Two days before transfection, GP2-293 cells were seeded at 2 million cells per 100 mm culture dish. For transfection, 5 micrograms of DNA + 2.5 micrograms of pMX-vsvg + OPTI-MEM were prepared in a total volume of 400 microliters in an Eppendorf tube (1). 22.5 microliters of Lipofectamine 2000 and 377.5 microliters of OPTI-MEM were added to an Eppendorf tube (2) to make a total volume of 400 microliters. After mixing the Eppendorf tube (1) and Eppendorf tube (2), a reaction was carried out at room temperature for 5 minutes. Afterwards, it was mixed with 7 ml of Dulbecco's Modified Eagle Medium (DMEM/High glucose media) without fetal bovine serum (FBS), and the mixture was treated to cells.

The first harvest of media was performed 24 hours after transfection, and 1 million mesenchymal stem cells to be transduced were seeded in 100 mm culture dishes on the same day. 48 hours after transfection, the media were secondly harvested, mixed with the media harvested a day before, and centrifuged at 1,300 rpm for 3 minutes. Afterwards, only the supernatant was collected, filtered using a 0.45 micrometer filter, and then centrifuged twice at 4,000 × g for 20 minutes using a 10 kilodalton Millipore Amicon filter. Cells to be transduced were treated with virus held on the filter together with 10 ml of Dulbecco's Modified Eagle Medium (DMEM/Low glucose media) and 10 microliters of Polyblene dissolved at 8 mg per ml. 24 hours after transduction, the medium was replaced with DMEM/Low Glucose Media.

### 2. Method of extracting EVs from mesenchymal stem cells

When the cells are about 90% confluent, washing was performed twice with PBS followed by replacement of medium with Dulbecco's Modified Eagle Medium (DMEM/Low glucose media) without fetal bovine serum (FBS). After 24 hours, the medium was obtained and centrifuged at 1,300 rpm for 3 minutes to remove cell debris. After collecting the supernatant again, filtration was followed in a size of 0.22 micrometer. The filtered medium was centrifuged three times at 4000 × g for 20 minutes using a 10 kilodalton Millipore Amicon filter. The obtained EVs were diluted with PBS to a total volume of 200 microliters. When extracting the processed EVs, the same method as above was proceeded, starting with washing twice with PBS two days after transduction.

### 3. Measurement of the concentration and size of extracted EVs

After mixing 10 microliters of the EV dilution solution distilled in 200 microliters in total and 990 microliters of PBS, the concentration of EV was measured via Nanoparticle tracking analysis (NTA).

### 4. Analysis of protein expression of EVs

1×10⁹ EVs were injected into one well of a PAGE gel, separated by electrophoresis, and then transferred to a nitrocellulose filter membrane. The filter membrane was reacted for 1 hour with TBST solution (Tris-buffered saline Triton × 100) in which 5% skim milk is dissolved, followed by a reaction for 18 hours with 5% bovine serum albumin (BSA) in which primary antibodies fibronectin (abcam), CD9 (santacruz), HSP70 (santacruz), GAPDH (santacruz) are diluted at a ratio of 1:500. After washing the filter membrane again with TBST three times for 8 minutes each, secondary antibodies antimouse-HRP and anti-goat-HRP were diluted in 5% skim milk at a ratio of 1:2500, and a reaction was followed for 1 hour. After washing the filter membrane three times with TBST for 8 minutes each, ECL solution was treated to measure the intensity of protein bands using Bio-Rad Image Lab software.

### 5. Analysis of an intracellular EV delivery efficiency

A cover glass was placed in a 35 mm culture dish, 0.1% gelatin was coated on it, and MSCs were seeded at 1×10⁵. The media used were Dulbecco's Modified Eagle Medium (DMEM/Low glucose media) and cultured in an incubator at 37°C in the presence of 5% carbon dioxide for one day. Additionally, the PKH26 staining kit was used to stain EVs with fluorescent dye. The 10^9 extracted exosomes were placed in a 100 kilodalton Millipore Amicon filter along with 400 microliters of PBS and centrifuged at 13,000 rpm for 20 minutes to concentrate EVs. 400 microliters of Diluent C solution in the PKH 26 dye kit was mixed with 0.8 microliters of PKH26 Dye, followed by a reaction for 1 minute. The product was reacted with concentrated EVs for 5 minutes. After 5 minutes, centrifugation was performed twice at 13,000 rpm for 20 minutes each. After centrifugation, the filtered solution was extracted with a pipette and transferred to a new 100 kilodalton Millipore Amicon filter, 400 microliters of PBS were added, and centrifugation was performed at 13,000 rpm for 20 minutes. PKH 26-stained EVs were obtained after centrifugation at 13,000 rpm for 1 minute by turning a 100 kilodalton Millipore Amicon filter over. 200 microliters of PBS was added to be stored in the refrigerator.

The next day, the media were replaced, and 1×10⁸ EVs stained with PKH26 were treated by mixing with the media in one 35 mm culture dish. Taken out after 3 hours of reaction from the incubator, the EVs were washed with PBS for 5 minutes and subjected to imaging using a fluorescence microscope or flow cytometry analysis to measure the EV transfer efficiency. When imaging using a fluorescence microscope, the EVs were treated with 4% paraformaldehyde for 5 minutes at room temperature and then washed three times with PBS for 1 minute each. A solution, in which 1 milligram of 4,6-diamidino-2-phenylindole (DAPI) per milliliter is dissolved, was diluted in PBS at a ratio of 1:500, reacted at room temperature for 1 minute, and then washed three times with PBS for 2 minutes each. The cover glass was turned over, placed on a slide glass, and then observed using a high-resolution automatic fluorescence microscope. When using a flow cytometer, cells were harvested using the medium after treatment with trypsin for 3 minutes. The obtained cells were washed three times with PBS, and the ratio of cells showing fluorescence was measured using a flow cytometer.

### <Example 1> Production of a Lamp2b protein overexpression vector in which RGD and Hep II domains of fibronectin are combined

Prepared was a vector capable of overexpressing a protein in which the RGD and Hep II domains of fibronectin are combined with Lamp2b. The amino acid sequences of each protein were linked, a linker protein, GGGSGGGS, was added between the Hep II domain and Lamp2b, and an overexpression vector having the pMX retrovirus vector as a backbone was constructed.

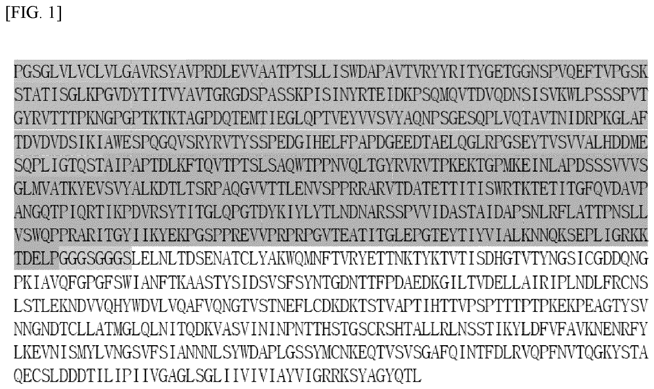

FIG. 1 shows amino acid sequences of RGD and Hep II domains of fibronectin and Lamp2b. Orange represents the signal peptide, green represents the sequence of the RGD domain, blue represents the sequence of the Hep II domain, yellow represents a sequence of a linker protein, and black represents a sequence of Lamp2b.

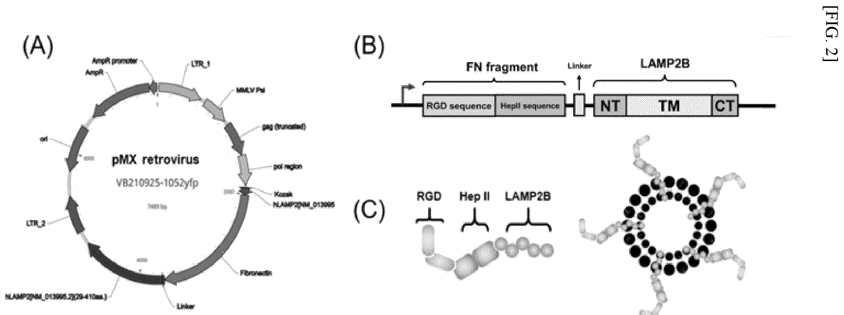

FIG. 2(A) shows a schematic diagram of a vector capable of expressing the RGD and Hep II domains of fibronectin combined with Lamp2b. FIG. 2(B) shows a schematic diagram visualizing a target protein in a vector, and FIG. 2(C) shows a schematic diagram illustrating a resulting processed EV.

### <Example 2> Analysis of the EV size

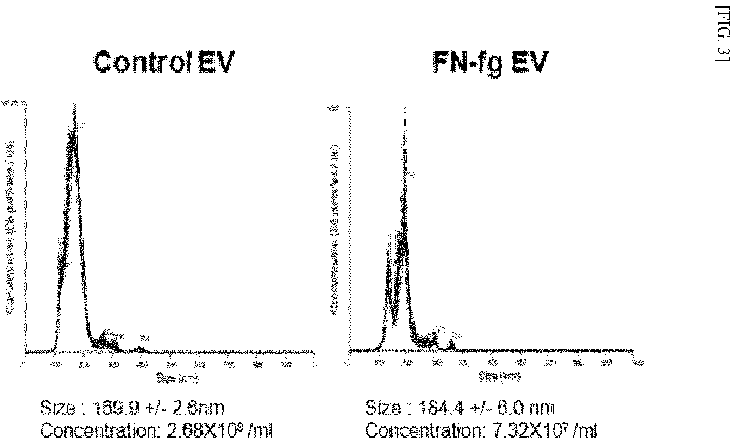

FIG. 3 shows results of measuring the size distribution of fibronectin domain-overexpressing EVs and a control group via NTA. As shown in Table 1, the control group was set as a pMXs mock vector.

**TABLE 1**

| Category | Gene transduction vector | Name |
|---|---|---|
| Control group | pMXs-Mock | Control EV |
| Experimental group | pMXs-FN-fg | FN-fg EV |

The results of measuring the size distribution of EVs showed that both the control group and the experimental group had similar size distributions.

### <Example 3> Analysis of protein expression of EVs

Fibronectin domain overexpression and EV marker gene expression in fibronectin domain-overexpressing EVs were identified by Western blot. Protein expression of the control and experimental group was compared as shown in Table 2.

**TABLE 2**

| Category | Gene transduction vector | Name |
|---|---|---|
| Control group | pMXs-Mock | Control Exo |
| Experimental group | pMXs-FN-fg | FN-fg Exo |

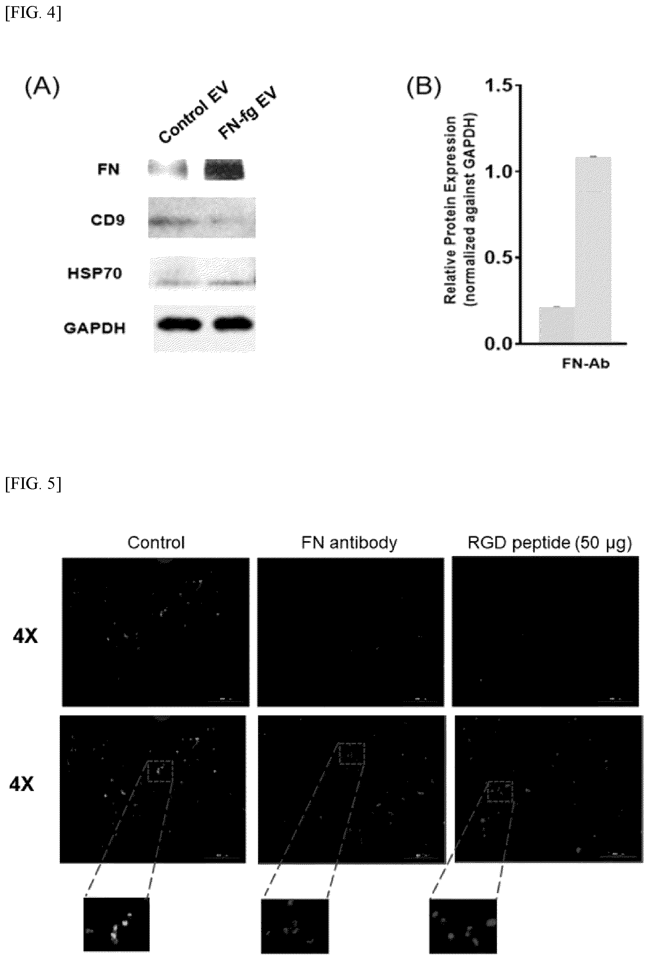

FIG. 4(A) shows results of identifying expression of fibronectin, EV marker genes (CD9, HSP70), and GAPDH in the control and experimental groups. The results showed that the expression of fibronectin increased in the experimental group compared to the control group. In addition, it was found that EV marker genes (CD9, SHP70) were expressed in both the control and experimental groups.

Normalization of the total protein amount of the comparison groups was proceeded through the expression level of GAPDH. FIG. 4(B) shows quantification of a band intensity of fibronectin in FIG. 4(A) using the Image J program. Normalization of the band intensity of fibronectin was performed based on the band intensity of GAPDH. In the result, it was found that fibronectin expression increased in the experimental group compared to the control group.

### <Example 4> Analysis of the intracellular delivery efficacy through fibronectin domain blocking

To determine whether intracellular EV delivery is mediated by fibronectin or RGD sequences, a comparison group was established as shown in Table 3, 1×10⁸ EVs were treated with 1 microliter of fibronectin antibody and 50 micrograms of RGD peptide, respectively followed by a reaction at 4°C for 12 hours, and then EVs were subjected to PKH67 staining through the method above and treated to cells. The uptake rate was determined by assessing the presence of green fluorescent EVs around the nuclei of cells stained with DAPI.

**TABLE 3**

| Category | Treatment | Name |
|---|---|---|
| Control group | - | Control |
| Experimental group 1 | Fibronectin antibody | FN antibody |
| Experimental group 2 | RGD peptide | RGD peptide |

FIG. 5 showed that when treated with the fibronectin antibody and RGD peptide, the EV delivery rate into the cell was significantly dropped compared to that of the control group with no addition. Thus, it was found that fibronectin and RGD peptide-binding receptors are involved in the EV delivery into cells.

### <Example 5> Analysis of the intracellular delivery efficiency of EVs overexpressing the fibronectin domain

To identify the intracellular delivery efficiency of fibronectin domain-overexpressing EVs, a comparative experiment was conducted by setting a comparison group as shown in Table 4. To identify the intracellular delivery efficiency, EVs fluorescently labeled with PKH26 were used, and the fluorescence intensity was measured using a fluorescence microscope.

**TABLE 4**

| Category | Treatment |
|---|---|
| Control group | None |
| Experimental group 1 | Control EV |
| Experimental group 2 | FN-fg EV |

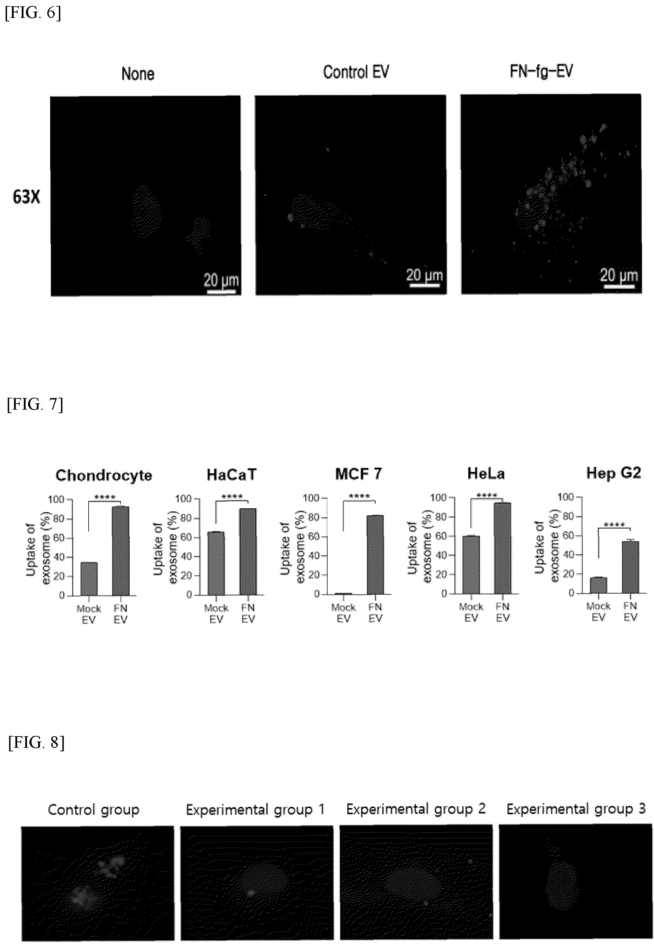

FIG. 6 shows results of identifying the intracellular delivery efficiency of EVs overexpressing the fibronectin domain. In the result, it was found through fluorescence imaging that the fluorescence intensity of experimental group 2 was higher than that of the control group and experimental group 1.

### <Example 6> Analysis of the intracellular delivery efficacy of EVs overexpressing the fibronectin domain in various types of cells

To determine whether fibronectin domain-overexpressing EVs increase the intracellular delivery efficiency in different cell types, the intracellular delivery efficiency was determined using different cell types. 1×10⁵ cells were treated with 1×10⁸ EVs stained with PKH26, and the transfer efficiency was identified through flow cytometry analysis.

**TABLE 5**

| Cell name | Cell type |
|---|---|
| Chondrocyte | Human chondrocyte (human-derived chondrocyte) |
| HaCaT | Human keratinocyte (human-derived keratinocyte) |
| MCF 7 | Mouse breast cancer (mouse-derived breast cancer cells) |
| HeLa | Human cervical cancer (human-derived cervical cancer cells) |
| Hep G2 | Human hepatocellular carcinoma (human-derived hepatic cancer cell) |

**TABLE 6**

| Category | Overexpression |
|---|---|
| Mock EV | - |
| FN EV | Fibronectin sequence (RGD domain + Hep II domain) |

In FIG. 7, it was determined that fibronectin domain-overexpressing EVs (FN EVs) showed remarkably increased intracellular delivery efficiency in various types of cells compared to Mock EVs, which were EVs with no overexpression. It was found that fibronectin-overexpressing EVs showed higher intracellular delivery efficiency than the control group not only in human-derived chondrocytes and keratinocytes, but also in breast cancer cells, cervical cancer cells, and liver cancer cells.

### <Example 7> Analysis of the intracellular delivery efficacy by blocking fibronectin domain of EVs overexpressing fibronectin

To determine whether the intracellular delivery of fibronectin-overexpressing EVs was mediated by the RGD sequence or Hep II domain 2, comparison groups were selected as shown in Table 5, and treatment was followed by 0.5 microliter of anti-RGD antibody for 1 × 10⁸ EVs in experimental group 1, and 0.5 microliter of anti-Hep II antibody for 1×10⁸ EVs in experimental group 2. In experimental group 3, 1×10⁸ EVs were reacted by 0.5 microliter of anti-RGD antibody and anti-Hep II antibody, respectively. After reacting at 4°C for 12 hours, EVs were subjected to PKH26 staining via the method above and treated to cells. The intracellular delivery efficiency was determined by the presence of red fluorescent EVs inside cells stained with DAPI.

**TABLE 7**

| Category | Treatment |
|---|---|
| Control group | - |
| Experimental group 1 | Anti-RGD antibody |
| Experimental group 2 | Anti-Hep II antibody |
| Experimental group 3 | Anti-RGD antibody + anti-Hep II antibody |

FIG. 8 shows that the intracellular delivery efficiency was significantly reduced in experimental group 3 treated with anti-RGD antibodies and anti-Hep II antibodies compared to the control group. In addition, the intracellular delivery efficiency was shown to be lowest in experimental group 3, which was treated with both antibodies simultaneously, compared to experimental groups 1 and 2 with treatment of either anti-RGD antibody or anti-Hep II antibody, respectively. Therefore, it was found that the intracellular delivery efficiency was highest when the RGD sequence and HepII domain were present simultaneously rather than being separate.

### <Example 8> Analysis of the intracellular delivery efficacy through comparison of EVs overexpressing Hep II domain and RGD domain separately and EVs expressing both domains

To determine whether the increase in the intracellular delivery efficiency of EVs depends on the simultaneous overexpression of the Hep II domain and the RGD sequence domain, EVs in which both domains are simultaneously overexpressed were compared with EVs in which the Hep II domain and the RGD sequence domain are individually overexpressed. 1×10⁵ cells were treated with 1×10⁸ PKH26-stained EVs. The intracellular delivery efficiency was identified by the presence of red fluorescent EVs around the cell nucleus stained with DAPI using high-resolution fluorescence microscopy.

**TABLE 8**

| Category | Overexpression |
|---|---|
| Control group | - |
| Experimental group 1 | RGD domain + Hep II domain |
| Experimental group 2 | Hep II domain |
| Experimental group 3 | RGD domain |

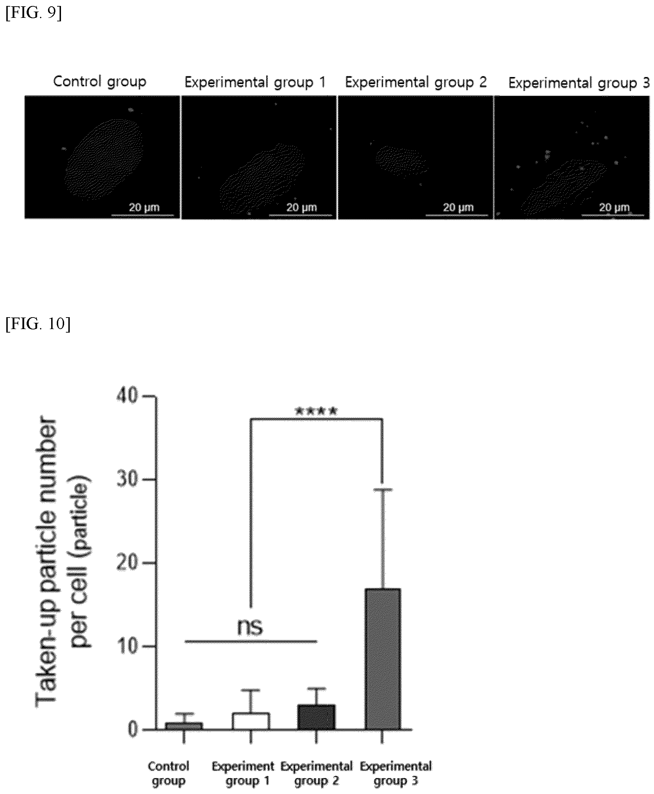

FIG. 9 shows results of identifying that the intracellular delivery efficiency of EVs was highest in experimental group 1, in which both domains are overexpressed, compared to the control group, which is an EV with no overexpression. In addition, in the comparison of experimental group 2 in which only the Hep II domain was overexpressed and experimental group 3 in which only the RGD domain was overexpressed, it was found that the intracellular delivery efficiency was significantly increased in experimental group 1 in which both domains were overexpressed. Therefore, it was determined that the intracellular delivery efficiency increased only when both domains are overexpressed, rather than when only one domain is overexpressed.

FIG. 10 shows the intracellular delivery efficiency of EVs by quantifying the red fluorescence intensity of FIG. 9. There was no statistical significance between the control, experimental group 2, and experimental group 3, but experimental group 1 was shown to have statistically significantly more EVs delivered compared to the other groups.

While a specific part of the present disclosure has been described in detail above, it is clear for those skilled in the art that this specific description is merely preferred example embodiments, and the scope of the present disclosure is not limited thereby. In other words, the substantial scope of the present disclosure is defined by the appended claims and their equivalents.

## Claims

1. An extracellular vesicle wherein a fibronectin fragment protein comprising a fibronectin RGD domain and a fibronectin Hep II domain is overexpressed on a membrane surface of the extracellular vesicle.

2. The extracellular vesicle of claim 1, wherein the fibronectin fragment protein is additionally fused a Lamp2b protein.

3. The extracellular vesicle of claim 1 or 2, wherein the fibronectin RGD domain is represented by an amino acid sequence of SEQ ID NO: 1, the fibronectin Hep II domain is represented by an amino acid sequence of SEQ ID NO: 2, and the Lamp2b protein is represented by an amino acid sequence of SEQ ID NO: 3.

4. The extracellular vesicle of claim 2, wherein the fibronectin fragment protein and the Lamp2b protein are linked by a linker.

5. The extracellular vesicle of claim 1 or 2, wherein the extracellular vesicle has a size of 100 to 200 nm.

6. A drug delivery composition comprising the extracellular vesicle of claim 1 or 2.

7. The drug delivery composition of claim 6, wherein the composition enhances a delivery efficiency of extracellular vesicles into cells.
